# EUROPÄISCHE PATENTSCHRIFT

(11) **EP 4 186 556 B1**
(45) Veröffentlichungstag und Bekanntmachung des Hinweises auf die Patenterteilung: **25.02.2026**
(21) Anmeldenummer: 21210433.5
(22) Anmeldetag: 25.11.2021
(51) Int. Cl.: A61M 39/22, A61M 5/145, A61M 5/142, A61M 5/168, A61M 5/24

(54) **MEDIZINISCHER HOCHDDRUCKINJEKTOR**
HIGH PRESSURE MEDICAL INJECTOR
INJECTEUR MÉDICAL HAUTE PRESSION

(43) Veröffentlichungstag der Anmeldung: 31.05.2023
(73) Patentinhaber: Medtron AG, 66128 Saarbrücken (DE)
(72) Erfinder: HANNO, Altmeyer, 66538 Neunkirchen (DE); KREBER, Stefan, 66113 Saarbrücken (DE); SCHWEIGMANN, Michael, 66482 Zweibrücken (DE)
(74) Vertreter: KBN IP Patentanwälte Partnerschaft mbB

(56) Entgegenhaltungen:
- US-A1- 2003 220 605
- US-A1- 2012 181 226
- US-A1- 2017 021 085
- US-A1- 2018 071 447

## Beschreibung

Die Erfindung betrifft einen medizinischen Hochdruckinjektor zur Injektion einer in einer Spritze bereitgestellten Flüssigkeit in einen Patienten, wobei der Injektor mindestens eine Spritzenhalterung mit zugeordnetem Antrieb für die gehalterte Spritze sowie Verbindungsleitungen für die Überleitung der Flüssigkeit aus der Spritze in einen Patientenschlauch und/oder zur Befüllung der in der Spritzenhalterung gehalterten Spritze aufweist.

Medizinische Hochdruckinjektoren der eingangs genannten Art sind bekannt und werden beispielsweise im Umfeld von Computertomografen (CT) oder Magnetresonanztomografen (MRT) verwendet, um ein Kontrastmittel und/oder andere Flüssigkeiten, wie Kochsalzlösung, im Rahmen einer entsprechenden bildgebenden Untersuchung mit hohem Druck von bis zu 83 bar in den Patienten zu injizieren. Ein Beispiel eines derartigen medizinischen Hochdruckinjektors ist in der DE 10 2004 032 970 A1 offenbart. Die in der Spritzenhalterung des Injektors gehalterten Spritzen, üblicherweise ein oder zwei derartige Spritzen werden über ein System von Verbindungsleitungen angeschlossen, welche einerseits eine Druckleitung für die Injektion der mindestens einen Flüssigkeit aus der Spritze in den Patienten, andererseits häufig auch mindestens eine Saugleitung umfassen, die vor der Injektion in den Patienten eine Befüllung der Spritze mit der entsprechenden Flüssigkeit aus einem entsprechenden Vorratsbehälter ermöglicht. Die bei der Befüllung der Spritze(n) bzw. dem Injizieren der Flüssigkeit in den Patienten gewünschten Strömungsrichtungen werden durch entsprechend vorgesehene Rückschlagventile gewährleistet. Ein Beispiel eines derartigen Systems von Verbindungsleitungen ist Gegenstand der DE 203 01 094 U1. Solche Verbindungsleitungen sind aufgrund der vorherrschenden Hygieneanforderungen Einwegartikel und müssen von entsprechend geschulten Fachkräften vor Untersuchungsbeginn aufwendig getauscht und angeschlossen werden, was arbeitsintensiv und fehleranfällig ist. Dabei muss insbesondere darauf geachtet werden, dass keine Luft oder andere Gasansammlungen in den Verbindungsleitungen oder den Spritzen enthalten sind, da eine Injektion in den Patienten unter Umständen lebensbedrohliche Komplikationen mit sich bringen würde. Des Weiteren definieren die bislang verwendeten Verbindungsleitungen stets ein entsprechend großes Volumen, welches mit Flüssigkeit aufzufüllen ist und nach Abschluss der Untersuchung gemeinsam mit den Verbindungsleitungen entsorgt wird, was verbesserungswürdig erscheint. Auch kommen passive Ventile zum Einsatz, mit denen eine Vordruckerzeugung in der Flüssigkeit zur Bolusschärfung nicht möglich ist.

Bei Anwendungen auf dem Gebiet der Dialyse ist es bereits bekannt, Leitungswege für die Führung des Blutkreislaufs und ggf. zugeführter Medikamente in einer auswechselbaren Steuerkassette anzuordnen, wozu auf die US 2018/071447 A1, US 2012/181226 A1, US 2017/021085 und US 2003/220605 A1 verwiesen wird. Die bekannten Steuerkassetten sind jedoch weder für Anwendungen mit einem medizinischen Hochdruckinjektor bestimmt noch geeignet, da sie über keine entsprechende Druckfestigkeit verfügen.

Aufgabe der Erfindung ist es, einen medizinischen Hochdruckinjektor der eingangs genannten Art dahingehend weiterzubilden, dass die Nachteile des Standes der Technik überwunden werden.

Zur Lösung der gestellten Aufgabe wird erfindungsgemäß ein medizinischer Hochdruckinjektor gemäß den Merkmalen des Patentanspruchs 1 vorgeschlagen.

Vorteilhafte Ausgestaltungen und Weiterbildungen der Erfindung sind Gegenstand der abhängigen Ansprüche.

Der erfindungsgemäße Vorschlag sieht vor, dass die Verbindungsleitungen in einer wechselbaren Steuerkassette ausgebildet sind und der Injektor eine entsprechende Kassettenhalterung mit einem öffen- und schließbaren Aufnahmeraum aufweist, in den die Steuerkassette wechselbar einsetzbar ist.

Erfindungsgemäß wird insoweit vorgesehen, die Verbindungsleitungen zumindest über einen größeren Abschnitt derselben, idealerweise vollständig in einer wechselbaren und in eine entsprechende Kassettenhalterung des Hochdruckinjektors einsetzbaren Steuerkassette auszubilden, sodass der Aufwand für den Anschluss der Verbindungsleitungen auf ein Minimum reduziert wird. Es ist erfindungsgemäß lediglich notwendig, eine solche Steuerkassette, die die Verbindungsleitungen aufweist, in die im Injektor vorgesehene Kassettenhalterung einzusetzen und mit der benötigten Anzahl an Spritzen zu verbinden und andererseits den Patientenschlauch an die Steuerkassette anzuschließen. Gegebenenfalls kann die Steuerkassette auch an entsprechenden Anschlüssen mit Vorratsbehältern der zu injizierenden Flüssigkeiten verbunden werden, um die Spritzen vor der Untersuchung in an sich bekannter Weise mit der Flüssigkeit aufzuziehen.

Die Steuerkassette ist dazu bevorzugt mit verschiedenen Anschlüssen ausgebildet, die beispielsweise als Teil einer Luer-Lock-Verbindung ausgestaltet sein können.

Erfindungsgemäß werden die für den Betrieb des medizinischen Hochdruckinjektors benötigten Verbindungsleitungen zumindest abschnittsweise, vorzugsweise vollständig in der wechselbaren Steuerkassette ausgebildet, sodass sie in der Steuerkassette vorkonfiguriert mit geringstmöglicher Länge und entsprechendem Minimalvolumen ausgebildet sind und nach dem Anschließen der Spritzen, des Patientenschlauchs und gegebenenfalls der Vorratsbehälter unmittelbar eine vollständige und korrekte Anschlusssituation für den sicheren Betrieb des erfindungsgemäßen Hochdruckinjektor vorliegt. Der Aufwand für das Ausbilden und den Anschluss der Verbindungsleitungen sowie die Gefahr von Bedienungsfehlern wird somit signifikant verringert.

Erfindungsgemäß umfasst die Steuerkassette einen mindestens zweiteiligen Aufbau und weist ein Hartteil und mindestens eine Membran auf. Im Hartteil werden in einer als Leitungsfläche ausgebildeten Oberfläche Leitungsabschnitte zur Ausbildung der Verbindungsleitungen in der Steuerkassette ausgebildet, indem sie beispielsweise nutartig in die Oberfläche eingefräst oder als nutartige Vertiefungen unmittelbar bei Formgebung des Hartteiles in diesem ausgebildet werden. Die solchermaßen mit den Leitungsabschnitten versehene und als Leitungsfläche bezeichnete Oberfläche des Hartteiles wird von einer darauf flüssigkeitsdicht aufgebrachten Membran begrenzt, d. h. die Membran bildet einen flüssigkeitsdichten Abschluss der im Hartteil ausgebildeten Leitungsabschnitte und überdeckt die Leitungsfläche einschließlich der darin eingebrachten Leitungsabschnitte.

Es ist darüber hinaus erfindungsgemäß vorgesehen, dass das Hartteil auf der der Leitungsfläche gegenüberliegenden und als Ventilfläche bezeichneten Oberfläche des Hartteiles Ventilabschnitte der Verbindungsleitungen aufweist, die in die Ventilfläche vorzugsweise als nutartige Vertiefungen eingebracht sind. Mittels einer auf die Ventilfläche flüssigkeitsdicht aufgebrachten weiteren Membran sind auch die Ventilabschnitte flüssigkeitsdicht abgedeckt und kommunizieren über durch das Hartteil von der Leitungsfläche bis zu Ventilfläche verlaufende Verbindungsbohrungen mit den Leitungsabschnitten im Bereich der Leitungsfläche. Gemeinsam mit den Leitungsabschnitten bilden die Ventilabschnitte die Verbindungsleitungen in der Steuerkassette aus.

Darüber hinaus ist die die Ventilabschnitte überdeckende Membran mittels einer von der Außenseite der Membran angreifenden Druckkraft elastisch auslenkbar, sodass sie als Ventil dienen kann und im entspannten Zustand den Strömungskanal freigibt, jedoch bei Aufbringen einer Druckkraft den Strömungskanal verengt oder vollständig verschließt. Dazu ist erfindungsgemäß die Kassettenhalterung mit in den Aufnahmeraum vorschiebbaren Stößeln ausgerüstet, die jeweils Ventilabschnitten eine eingesetzten Steuerkassette zugeordnet sind und von einer entsprechenden Steuerung mittels zugeordneter linearer Antriebe in den Aufnahmeraum vorschiebbaren bzw. in entgegengesetzter Richtung aus dem Aufnahmeraum zurückziehbar sind. Mittels eines in den Aufnahmeraum vorgeschobenen und auf einen Ventilabschnitt drückenden Stößels kann somit selektiv der jeweilige Ventilabschnitt in Abhängigkeit von der ausgeübten Druckkraft zumindest teilweise oder auch vollständig verschlossen werden und nach zurückziehen des Stößels in entgegengesetzter Richtung vollständig freigegeben werden. Auf diese Weise können in der erfindungsgemäß vorgesehenen Steuerkassette des medizinischen Hochdruckinjektors nicht nur die Verbindungsleitungen, sondern mittels der elastisch auslenkbaren Membran über den Ventilabschnitten auch die für die Durchflusssteuerung erforderlichen Ventile ausgebildet werden und somit die bislang in den Verbindungsleitungen vorgesehenen Rückschlagventile ersetzt und in der Steuerkassette vorgegeben werden.

Nach einem Vorschlag der Erfindung ist vorgesehen, dass das Hartteil beispielsweise aus einem spritzgegossenen Kunststoff und die Membranen jeweils aus einer mit dem Hartteil verschweißbaren Kunststofffolie gebildet sind, sodass die gewünschte flüssigkeitsdichte Verbindung zwischen Hartteil und Membran beispielsweise durch eine Laserverschweißung erfolgen kann. Mit einer solchen Ausgestaltung kann die erfindungsgemäße Steuerkassette rationell in hohen Stückzahlen gefertigt werden, was insbesondere dem Umstand Rechnung trägt, dass die Steuerkassette, welche die Ventile und Verbindungsleitungen enthält, als Einwegteil nach Abschluss eines Untersuchungszyklus des Hochdruckinjektors ausgetauscht und verworfen werden muss.

Nebenbei bemerkt schließt die Verwendung der Steuerkassette als Einwegteil sowohl eine einmalige Nutzung bei lediglich einem Patienten wie auch eine Nutzung über einen begrenzten Zeitraum, zum Beispiel einen Tag oder 24 Stunden für eine entsprechende Anzahl an Patienten ein.

Es versteht sich, dass bei entsprechendem Bedarf auch in der Ventilfläche Leitungsabschnitte ausgebildet sein können, wie auch im Bereich der Leitungsfläche Ventilabschnitte ausgebildet werden können.

Um die Ventilfunktion in der Steuerkassette mittels der elastisch auslenkbaren Membran bewirken zu können, sind die in den Aufnahmeraum vorschiebbaren Stößel beispielsweise pneumatisch oder mittels elektrischer Linearantriebe betätigbar und so in der Kassettenhalterung relativ zur Einbaulage der Steuerkassette angeordnet, dass sie auf einen zugeordneten Ventilabschnitt der in den Aufnahmeraum eingesetzten Steuerkassette unter Erzeugung einer Druckkraft an einer gewünschten Position der Steuerkassette pressbar sind. Abhängig vom Verlauf der Leitungs- und Ventilabschnitte in der Steuerkassette können somit durch entsprechende Anordnung der Stößel die Ventile an geeigneten Positionen in der Steuerkassette vorgesehen werden.

Nach einem weiteren Vorschlag der Erfindung können die in der Steuerkassette ausgebildeten Verbindungsleitungen auch eine Einrichtung zur Abscheidung von Gasblasen aus einer durch die Verbindungsleitungen strömenden Flüssigkeit umfassen, um der versehentlichen Einleitung eines Gasvolumens in den Patientenschlauch und letztlich in den Patienten entgegenzuwirken.

Eine solche Einrichtung zur Abscheidung von Gasblasen, die in die Steuerkassette integrierbar ist, kann beispielsweise einen kreisbogenförmig verlaufenden Kanalabschnitt der Verbindungsleitung umfassen, dessen Radius sich in einer vertikal verlaufenden Ebene erstreckt, wobei am höchsten Punkt des Bereichs eine Gasaustrittsöffnung vorgesehen ist und der Kanal sich darüber hinaus in Strömungsrichtung der Flüssigkeit betrachtet verjüngt. Mit einer solchen Einrichtung ist eine zuverlässige Abscheidung der etwaig anfallenden Gasvolumina im Flüssigkeitsstrom möglich. Es ist insbesondere vorgesehen, die Einrichtung zur Abscheidung der Gasblasen in den in Durchströmrichtung durch die Steuerkassette aufeinanderfolgenden Verlauf der Leitungsabschnitte zu integrieren, d. h. ein Leitungsabschnitt kommuniziert mit einem Eingang des Kanals der Einrichtung und an den Ausgang des Kanals schließt sich ein weiterer Leitungsabschnitt an, wobei sowohl eingangsseitig als auch ausgangsseitig ein entsprechender Ventilabschnitt vorgesehen sein kann, um den Durchfluss zu steuern.

Nach einem weiteren Vorschlag der Erfindung kann die Steuerkassette auch eine optische Detektionseinrichtung zum Detektieren von Gasblasen in einem Leitungsabschnitt umfassen, vorzugsweise in demjenigen Leitungsabschnitt, die zum Patientenschlauch führt.

Eine solche Detektionseinrichtung kann nach einem weiteren Vorschlag der Erfindung zwei an gegenüberliegenden Positionen zu der zu überwachenden Verbindungsleitung angeordnete Umlenkprismen aufweisen, von denen eines von einer in der Wandung des Aufnahmeraumes angeordneten Lichtquelle beleuchtet wird und den Lichtstrahl durch den überwachten Leitungsabschnitt zum gegenüberliegenden Umlenkprisma leitet und letzteres den auftreffenden Lichtstrahl zu mindestens einem in der Wandung des Aufnahmeraumes angeordneten Sensor umleitet. Die Umlenkprismen können beispielsweise bei Herstellung des Hartteiles integral in diesem ausgebildet werden, sodass sie an einer definierten Position der Steuerkassette angeordnet und mit entsprechenden Lichtquellen und Sensoren in einem Strahlengang zusammenwirken, welche ortsfest im Aufnahmeraum des erfindungsgemäßen Hochdruckinjektors angeordnet sind. Somit wird erfindungsgemäß bei Einsetzen der Steuerkassette in den Aufnahmeraum zugleich auch die korrekte Positionierung der Umlenkprismen zur Lichtquelle sowie den Sensoren bewirkt.

Nach einem weiteren Vorschlag der Erfindung kann die Steuerkassette bereichsweise mit einer Heizeinrichtung für die durch die Verbindungsleitungen geleitete Flüssigkeit ausgerüstet sein. Eine solche Heizeinrichtung zur Beheizung der durch die Verbindungsleitungen geleitete Flüssigkeit ermöglicht es zum einen, die Flüssigkeit auf eine für den Patienten angenehme Temperatur, insbesondere dessen Körpertemperatur zu erwärmen, zum anderen kann insbesondere Kontrastmittel, welches häufig bei Raumtemperatur relativ dickflüssig ist, auf eine für gewünschte hohe Flowraten geeignete Temperatur erwärmt werden.

Die Heizeinrichtung kann nach einem Vorschlag der Erfindung beispielsweise von einer bereichsweise auf die Steuerkassette aufgebrachten strahlungsabsorbierenden Beschichtung und einer zugeordneten Lichtquelle im Aufnahmeraum gebildet werden, beispielsweise einem entsprechenden LED-Strahler.

Hierzu kann vorgesehen sein, einen oder mehrere Leitungsabschnitte in der Steuerkassette mit einem mäanderförmigen Verlauf zu führen und beispielsweise auf eine der Membranen in einem vorbestimmten Sektor eine entsprechend eingefärbte Folie aufzubringen oder auch das Hartteil entsprechend einzufärben. Neben der Vorwärmung der zu injizierenden Flüssigkeiten bietet die Ausgestaltung der Heizeinrichtung mit einem Strahler und einer strahlungsabsorbierenden Beschichtung darüber hinaus den Vorteil, dass durch An- bzw. Abschaltung des Strahlers eine schnelle Feinregulierung der Temperatur ohne Hysterese bewirkt werden kann.

Nach einer weiteren Ausgestaltung der Erfindung ist vorgesehen, dass die Steuerkassette und mindestens eine Spritze, vorzugsweise zwei Spritzen, zu einem wechselbaren Modul zusammengefasst und miteinander verbunden sind, sodass sie gemeinsam in den Aufnahmeraum und die Spritzenhalterung einsetzbar sind. Ein solcher fester Verbund aus Spritze(n) und Steuerkassette erleichtert die Handhabung des erfindungsgemäßen Hochdruckinjektors noch weiter, da kein Anschluss der Steuerkassette an die beiden Spritzen mehr vorgenommen werden muss. Die Spritzen können darüber hinaus je nach Spritzentyp in vertikaler oder eher horizontaler Orientierung in den Aufnahmeraum des erfindungsgemäßen Hochdruckinjektors eingesetzt werden.

Der korrekte Einsatz der Steuerkassette in den Aufnahmeraum des erfindungsgemäßen Hochdruckinjektors kann nach einem weiteren Vorschlag der Erfindung dadurch sichergestellt werden, dass der Aufnahmeraum und/oder die Steuerkassette mit Mitteln zum lagerichtigen Einsetzen der Steuerkassette in den Aufnahmeraum ausgerüstet sind. Dies kann beispielsweise durch im Aufnahmeraum vorgesehene vorstehende Stifte bewirkt werden, die entsprechende Ausnehmungen der Steuerkassette eingreifen, sofern diese in der korrekten Orientierung in den Aufnahmeraum eingesetzt wird. Es versteht sich, dass diese Anordnung von Stiften und Ausnehmungen auch umgekehrt gewählt werden kann.

Schließlich kann die Steuerkassette des erfindungsgemäßen Hochdruckinjektors nach weiterer Ausgestaltung der Erfindung auch mit Identifizierungsmitteln ausgebildet sein, welche vom Hochdruckinjektor bei in den Aufnahmeraum eingesetzter Steuerkassette auslesbar sind. Beispiele derartiger Identifizierungsmittel umfassen einen Mikrochip auf der Steuerkassette, eine PIN-Kodierung, einen Barcode, einen RFID-Chip etc. Mit einem solchen Identifizierungsmittel können nicht nur Originalitätsnachweise, sondern auch Haltbarkeitsdaten, Anwendungsdauern, Injektionsparameter etc. übergeben oder auf dem Identifizierungsmittel abgespeichert werden.

Weitere Ausgestaltungen und Einzelheiten der Erfindung werden nachfolgend anhand der ein Ausführungsbeispiel darstellende Zeichnung erläutert. Es zeigen:
- Figur 1: eine Steuerkassette eines erfindungsgemäßen Hochdruckinjektors in Explosionsdarstellung;
- Figur 2: die Vorderansicht auf das Hartteil der Steuerkassette gemäß Figur 1;
- Figur 3: die Rückansicht auf das Hartteil der Steuerkassette gemäß Figur 1 ;
- Figur 4: die in den erfindungsgemäßen Hochdruckinjektor eingesetzte Steuerkassette;
- Figur 5: die Anordnung gemäß Figur 4 in einer geschnittenen Seitenansicht;
- Figur 6a: die Aufsicht auf eine Detektionseinrichtung gemäß Figur 2 in vergrößerter Darstellung;
- Figur 6b: die Detektionseinrichtung gemäß Figur 6a aus einer um 90° gedrehten Perspektive.

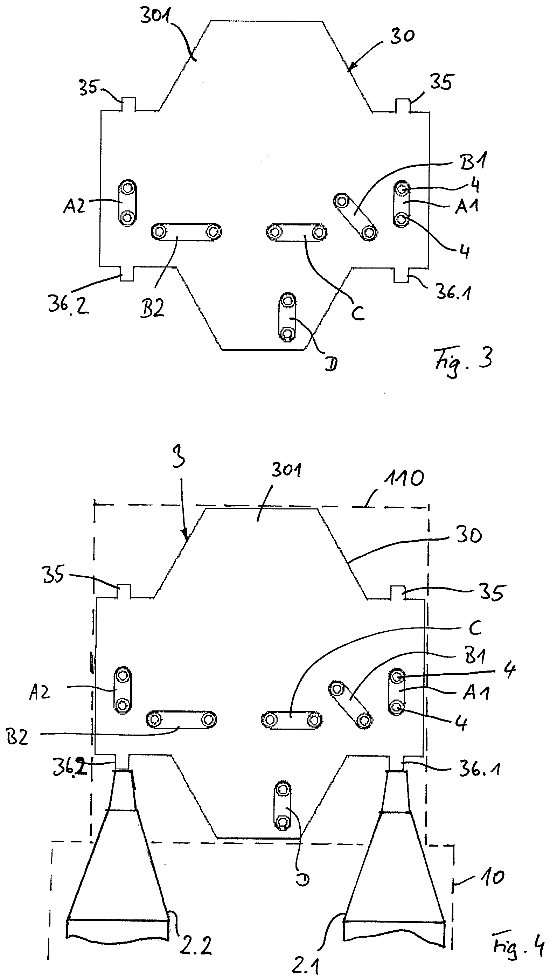

Aus den Figuren ist ein medizinischer Hochdruckinjektor zum Beispiel für den Einsatz in Kombination mit einem CT- oder MRT-Gerät ersichtlich, der gemäß der Darstellung in Figur 4 eine Spritzenaufnahme 10 für zwei Spritzen 2.1 und 2.2 nebst entsprechenden, hier nicht dargestellten Antrieben für die Spritzenbetätigung und einer entsprechenden Steuerung umfasst. Die Spritze 2.1 kann beispielsweise mit einem Kontrastmittel und die Spritze 2.2 mit Kochsalzlösung gefüllt sein oder werden.

Die jeweiligen Spritzenöffnungen der Spritzen 2.1 und 2.2 sind an entsprechende Anschlüsse 36.1, 36.2 einer Steuerkassette 3 angeschlossen, die ihrerseits in einem entsprechenden Aufnahmeraum 110 einer Kassettenhalterung 11 des Hochdruckinjektors eingesetzt ist. Der Aufnahmeraum 110 grenzt somit unmittelbar an die Spritzenaufnahme 10 an.

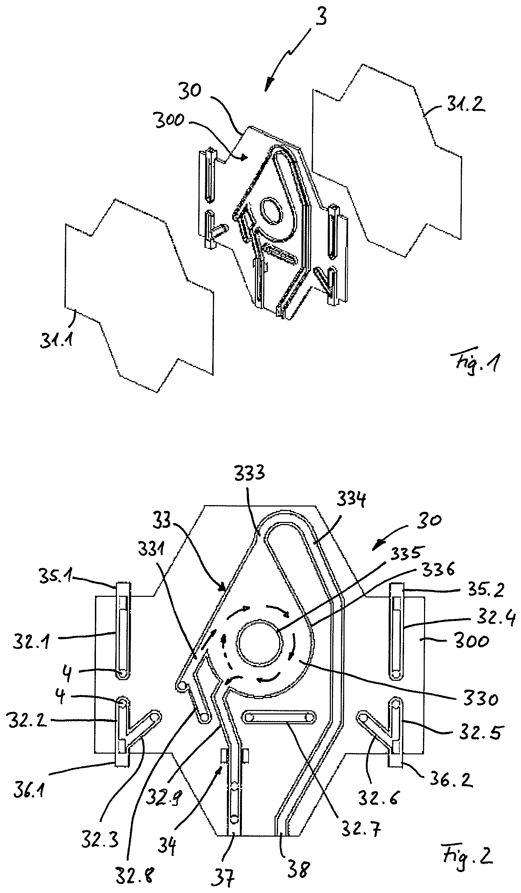

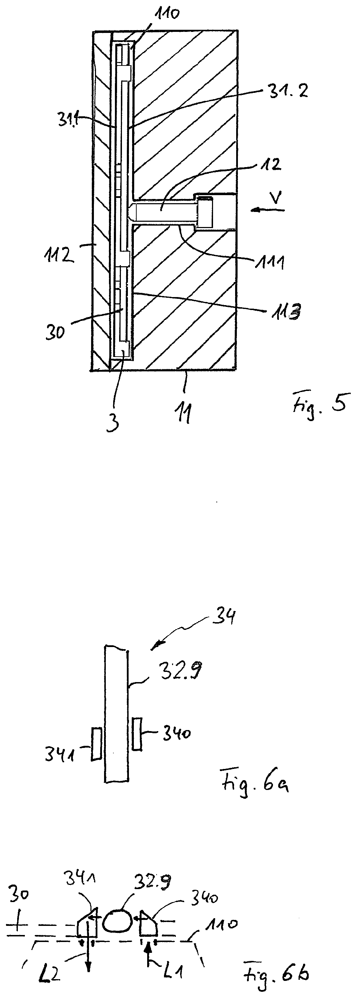

Wie insbesondere aus der Darstellung gemäß Figur 5 ersichtlich, ist der in der Kassettenhalterung 11 ausgebildete Aufnahmeraum 110 mit einer entsprechenden Tür 112 verschließbar, wobei die Dimensionierung des Aufnahmeraumes 110 so gewählt ist, dass die Steuerkassette 3 lediglich in einer vorgegebenen Orientierung in den Aufnahmeraum 110 eingesetzt werden kann und nach Verschließen der Tür 112 vorderseitig an der Tür 112 und rückseitig an der Wand 113 des Aufnahmeraumes 110 abgestützt wird. Die Tür 112 kann gleichermaßen auch die Spritzenaufnahme 10 überdecken und verschließen. Die Orientierung ist so gewählt, dass die aus der Figur 2 ersichtliche Oberfläche der Steuerkassette 3 an der Tür 112 und die aus der Figur 3 ersichtliche Oberfläche der Steuerkassette 3 an der Wand 113 des Aufnahmeraumes 110 anliegt.

In der nachfolgend noch näher beschriebenen Weise sind sämtliche Verbindungsleitungen zum Betrieb des Hochdruckinjektors innerhalb der Steuerkassette 3 ausgebildet, was in näheren Einzelheiten aus den Figuren 1-3 ersichtlich ist.

Die Steuerkassette 3 weist einen mehrteiligen Aufbau auf und umfasst ein zentral angeordnetes Hartteil 30 aus beispielsweise spritzgegossenem Kunststoff, auf dessen beide gegenüberliegende Oberflächen jeweils eine Membran 31.1, 31.2 aus einer elastisch dehnbaren Kunststofffolie aufgelegt und flüssigkeitsdicht mit dem Hartteil 30 zum Beispiel mittels Laserschweißung verschweißt ist.

Wie insbesondere aus der Darstellung gemäß Figur 2 ersichtlich, ist das Hartteil 30 im Bereich seiner von der Membran 31.1 überdeckten Oberfläche, die nachfolgend als Leitungsfläche 300 bezeichnet wird, mit einer Vielzahl von nutartig eingebrachten Leitungsabschnitten 32.1, 32.2, 32.3, 32.4, 32.5, 32.6, 32.7, 32.8, 32.9 sowie einer Einrichtung 33 zur Abscheidung von Gasblasen ausgebildet, die nachfolgend erläutert werden.

Bei Ausbildung des Hartteiles 30 aus einem spritzgegossenen Kunststoff können diese Leitungsabschnitte 32.1, 32.2, 32.3, 32.4, 32.5, 32.6, 32.7, 32.8, 32.9 und/oder die Einrichtung 33 durch entsprechende Formgebung der verwendeten Spritzgussform unmittelbar eingebracht werden, alternativ können Sie auch aus einem Vollmaterial zum Beispiel spanabhebend ausgearbeitet werden.

Ferner erkennt man im Bereich des Außenumfanges des Hartteiles 30 neben den bereits erwähnten Anschlüssen 36.1 und 36.2 für die Verbindung der Spritzen 2.1 und 2.2 weitere am gegenüberliegenden Rand ausgebildete Anschlüsse 35.1 und 35.2, die wie die Anschlüsse 36.1 und 36.2 als Teil eines Luer-Lock-Verbinders ausgebildet sein können.

Die Anschlüsse 35.1 und 35.2 dienen dazu, an die Steuerkassette 3 bzw. deren Hartteil 30 Zuleitungen von nicht dargestellten Vorratsbehältern für Kontrastmittel und Kochsalzlösung anzuschließen, um die an die Anschlusselemente 36.1 und 36.2 anschließbaren Spritzen 2.1 und 2.2 befüllen zu können.

Die Steuerkassette 3 wird solchermaßen orientiert in den Aufnahmeraum 110 der Kassettenaufnahme 11 eingelegt, dass die von der Membran 31.1 überdeckte Leitungsfläche bei geschlossener Tür 112 an dieser anliegt.

Auf der der Leitungsfläche 300 gegenüberliegenden Oberfläche, die in näheren Einzelheiten auch aus der Figur 3 ersichtlich ist und nachfolgend als Ventilfläche 301 bezeichnet wird, sind ebenfalls an definierten Positionen nutartige Vertiefungen eingebracht, die Ventilabschnitte A1, A2, B1, B2, C und D ausbilden und über jeweils im Endbereich der Ventilabschnitte A1, A2, B1, B2, C, D ausgebildete und aus der Ventilfläche 301 durch das Hartteil 30 hindurch bis in die gegenüberliegende Leitungsfläche 300 verlaufende Bohrungen 4 mit dort ebenfalls in den Bohrungen 4 endenden Leitungsabschnitten (32.1, 32.2, 32.3, 32.4, 32.5, 32.6, 32.7, 32.8, 32.9) kommunizieren und insoweit gemeinsam die Verbindungsleitungen in der Steuerkassette 3 ausbilden.

So wird zum Befüllen der an die Anschlüsse 36.1 und 36.2 angeschlossenen Spritzen 2.1 und 2.2 jeweils eine Verbindungsleitung ausgehend von den Anschlusselementen 35.1 und 35.2 ausgebildet. Die von dem nicht dargestellten Vorratsbehälter an dem Anschluss 35.1 anliegende Flüssigkeit gelangt zunächst über den Strömungskanal 32.1 und am Ende desselben vorgesehene Bohrung 4 auf die gegenüberliegende Ventilfläche 301 gemäß Figur 3 in den dort ausgebildeten Kanal A1. Von dort führt eine weitere Bohrung 4 zurück in die Leitungsfläche 300 in einen Strömungskanal 32.2 und von dort über das Anschlusselement 36.1 in die zugeordnete Spritze 2.1. Gleichermaßen gelangt am Anschluss 35.2 anliegende Flüssigkeit über einen Strömungskanal 32.4 und eine am Ende vorgesehene Bohrung 4 in die Ventilfläche 301 und einen dortigen Ventilabschnitt A2 und über eine weitere Bohrung 4 zurück auf die Leitungsfläche 300 und den dortigen Ventilabschnitte 32.5, der letztlich zum Anschluss 36.2 für die Spritze 2.2 führt. Insoweit können die Spritzen 2.1 und 2.2 durch die Steuerkassette 3 hindurch über die darin ausgebildeten und vorangehenden erläuterten Verbindungsleitungen mit der jeweils gewünschten Flüssigkeit befüllt werden.

Der eigentliche Injektionsvorgang des Hochdruckinjektors aus den Spritzen 2.1 bzw. 2.2 in Richtung auf den Patienten erfolgt über einen hier nicht dargestellten Patientenschlauch, der an einem entsprechenden Port 37 der Steuerkassette 3 im Hartteil 30 angeschlossen wird. Ausgehend vom Anschlusselement 36.1 gelangt die zu injizierende Flüssigkeit aus der Spritze 2.1, beispielsweise Kontrastmittel, zunächst über einen Strömungskanal 32.3 und den in der gegenüberliegenden Ventilfläche 301 des Hartteiles 30 ausgebildeten Ventilabschnitt B1 in einen Zulauf 331 der nachfolgend noch näher erläuterten Einrichtung zur Gasabscheidung 33, den die Flüssigkeit anschließend über den Leitungsabschnitt 32.9 in Richtung des für den Patientenschlauch vorgesehenen Port 37 verlässt.

Gleichermaßen kann die aus der Spritze 2.2 zu injizierende Flüssigkeit über das zugehörige Anschlusselement 36.2 und die Leitungsabschnitte 32.6 auf den auf der gegenüberliegenden Ventilfläche 301 des Hartteiles 30 ausgebildeten Ventilabschnitt B2 in den Leitungsabschnitt 32.7 überführt werden und anschließend über den erneut auf der gegenüberliegenden Ventilfläche 301 des Hartteiles 30 ausgebildeten Ventilabschnitt C in den Leitungsabschnitten 32.8 und von dort in den Zulauf 331 der Einrichtung 33 gefördert werden, um abschließend über den Leitungsabschnitt 32.9 zum Port 37 zu gelangen, an den der Patientenschlauch angeschlossen wird.

Der jeweilige Wechsel der Flüssigkeit von der einen Oberfläche des Hartteiles gemäß Figur 2 auf die andere Oberfläche des Hartteiles gemäß Figur 3 erfolgt jeweils über die das Hartteil 30 durchsetzenden Durchgangsbohrungen 4, die mit den Leitungsabschnitten 32.1, 32.2, 32.3, 32.4, 32.5, 32.6, 32.7, 32.8, 32.9 im Bereich der Leitungsfläche 330 und den Ventilabschnitten H1, H2, B1, B2, C und D im Bereich der gegenüberliegenden Ventilfläche 301 kommunizieren.

Um den Durchfluss der Flüssigkeiten durch die Steuerkassette 3 in der vorangehend beschriebenen Weise vorzugeben, müssen die jeweils gewünschten Verbindungsleitungen freigegeben und die übrigen, nicht benötigten Verbindungsleitungen verschlossen werden. Beispielsweise wird beim Befüllen der Spritze 2.1 der Ventilabschnitt A1 geöffnet, wohingegen der Ventilabschnitt B1, der in Richtung des Ports 37 für den Patientenschlauch führt, verschlossen wird. Andererseits wird beim Injizieren von Flüssigkeit aus der Spritze 2.1 in Richtung des Ports 37 der Ventilabschnitt B1 geöffnet und der Ventilabschnitt A1 geschlossen, um den Strömungsweg zum Anschluss 35.1 abzuschalten. Gleiches gilt auch für die Verbindungsleitungen zwischen den Anschlüssen 35.2 und 36.2

Wie insbesondere aus der Darstellung gemäß Figur 5 ersichtlich, wird dazu die Steuerkassette 3 in die entsprechende Kassettenhalterung 11 des Hochdruckinjektors eingelegt, die den Aufnahmeraum 110 für die Steuerkassette 3 aufweist und nach dem Einlegen einer Steuerkassette 3 mittels der Tür 112 verschlossen werden kann. Die im Bereich beider Oberflächen des Hartteiles 30 der Steuerkassette 3 aufgebrachten Membranen 31.1 und 31.2 erfahren somit entlang der Wand 113 des Aufnahmeraumes 110 sowie der Innenoberfläche der Tür 112 eine vollflächige Abstützung, sodass sie auch hohen Drücken der in den Verbindungsleitungen des Hartteiles 30 geführten Flüssigkeit standhalten können.

Darüber hinaus sind in der Kassettenhalterung 11 in entsprechenden Bohrungen 111 eine Vielzahl von Stößeln 12 angeordnet, die beispielsweise pneumatisch in einer Pfeilrichtung V gemäß Figur 5 auf die benachbart angeordnete Membran 31.2 gepresst werden können, welche die aus der Figur 3 ersichtliche Ventilfläche 301 des Hartteiles 30 bedeckt, in der die Ventilabschnitte A1, A2, B1, B2, C und D ausgebildet sind.

Jeder dieser Stößel 12 ist so angeordnet, dass er bei korrekt in den Aufnahmeraum 110 eingesetzter Steuerkassette 3 exakt über einem der genannten Ventilabschnitte A1, A2, B1, B2, C oder D positioniert ist, sodass durch Vorschub des Stößels 12 gemäß Pfeilrichtung V der Strömungsquerschnitt durch den jeweiligen Ventilabschnitt A1, A2, B1, B2, C oder D aus einer Freigabeposition in eine diesen Ventilabschnitt verengende oder verschließende Position verändert werden kann, indem die angrenzende Membran 31 durch die vom Stößel 12 ausgehende Druckkraft elastisch in Richtung des Hartteiles 30 ausgelenkt wird. Auf diese Weise können die Ventile zur Steuerung des Durchflusses durch die einzelnen Verbindungsleitungen ausgebildet werden.

Wie vorangehend bereits erläutert, strömt die aus der Spritze 2.1 bzw. 2.2 injizierte Flüssigkeit auf ihrem Weg durch die Steuerkassette 3 aus dem Leitungsabschnitten 32.3 und in Ventilabschnitt B1 bzw. über die Leitungsabschnitten 32.6, 32.7 und 32.8 unter Zwischenschaltung der Ventilabschnitte B2 und C über einen gemeinsamen Zulauf 331 in die Einrichtung 33 zur Abscheidung von Gasblasen, die in den Verlauf der aus Leitungsabschnitten 32.1, 32.2, 32.3, 32.4, 32.5, 32.6, 32.7, 32.8, 32.9 und Ventilabschnitten A1, A2, B1, B2, C oder D gebildeten Verbindungsleitungen integriert ist. Die Abscheidung von Gasblasen aus der durchströmenden Flüssigkeit wird dadurch bewirkt, dass diese ausgehend vom Zulauf 331 in einen kreisbogenförmig verlaufenden Kanalabschnitt 330 gezwungen wird, der von einer äußeren Begrenzungswand 336 und einer inneren Begrenzungswand 335 definiert wird und sich überdies in der mit Pfeilen dargestellten Strömungsrichtung in seiner lichten Höhe verjüngt. Dies hat zur Folge, dass große mitgeführte Gasblasen bereits beim Umlenken des aus dem Zulauf 331 zugeführten Flüssigkeitsstroms in die kreisbogenförmige Bahn 330 absondern und die Einrichtung 33 über eine am höchsten Punkt angeordnete Gasaustrittsöffnung 333 verlassen. Sie werden insoweit getrennt von der Flüssigkeit über eine Ableitung 334 abgeführt und über einen Port 38 an eine entsprechende, hier nicht dargestellte Sammeleinrichtung übergeben.

Kleinere Gasblasen hingegen sammeln sich vornehmlich im Bereich der kreisförmigen Innenoberfläche 335 an und steigen von dort allmählich ebenfalls in Richtung des am höchsten Punkt angeordneten Gasaustritts 333 auf und können gesondert von der Flüssigkeit abgeführt werden.

Die von Gaseinflüssen befreite Flüssigkeit hingegen verlässt die Einrichtung 33 über den als Auslauf dienenden Leitungsabschnitt 32.9 in Richtung des am Port 37 angeschlossenen Patientenschlauchs. Der Leitungsabschnitt 32.9 kann im Bereich der gegenüberliegenden Ventilfläche ebenfalls über einen in den Strömungsweg integrierten Ventilabschnitt D mit einem entsprechenden Stößel 12 geschlossen oder freigegeben werden.

Es hat sich im Rahmen der Erfindung gezeigt, dass durch eine solche Ausgestaltung der Einrichtung 33 eine besonders zuverlässige Abscheidung von etwaigen in der Flüssigkeit enthaltenen Gasblasen durchgeführt werden kann, wobei die Abscheidewirkung umso höher ist, je höher die Strömungsgeschwindigkeit der Flüssigkeit ist. Die Strömungsgeschwindigkeiten bei einem Hochdruckinjektor sind angesichts anliegender Betriebsdrücke von bis zu 83 bar üblicherweise besonders hoch. Es kann im Übrigen vorkommen, dass Teilmengen der auf die Kreisbahn gezwungenen Flüssigkeit nicht unmittelbar in den Auslauf 332 übertreten, sondern noch einen oder gegebenenfalls auch mehrere weitere Umläufe durch die Einrichtung 33 auf der Kreisbahn vollführen. Dies ist jedoch unschädlich und steigert lediglich die Abscheidungswirkung.

Zur weiteren Erhöhung der Sicherheit gegen unerwünschte Gaseinschlüsse in der dem Patientenschlauch zugeführten Flüssigkeit ist überdies im Leitungsabschnitt 32.9 eine Detektionseinrichtung 34 vorgesehen, die in näheren Einzelheiten aus den Figuren 6a und 6b ersichtlich ist.

Diese Detektionseinrichtung 34 umfasst zwei beidseits des vorzugsweise transparent ausgeführten Leitungsabschnittes 32.9 angeordnete Umlenkprismen 340, 341. Eine im Bereich der Tür 112 oder der Wand 113 des Aufnahmeraumes 110 angeordnete Lichtquelle sendet einen Lichtstrahl L1 über eine entsprechende Öffnung in das Umlenkprisma 340, welches den Lichtstrahl um 90° gedreht durch den zu überwachenden Leitungsabschnitt 32.9 hindurch auf das weitere Umlenkprisma 341 im Bereich der gegenüberliegenden Seite des Leitungsabschnittes 32.9 richtet. Von dort wird der Lichtstrahl erneut um 90° gedreht über eine Öffnung im Aufnahmeraum 110 als Lichtstrahl L2 auf entsprechende, in der Kassettenhalterung 11 angeordnete Sensoren gerichtet, sodass eine Überwachung des Leitungsabschnittes 32.9 ermöglicht ist. Durch Anordnung mehrerer Sensoren bzw. Dioden ist eine konkrete Auswertung ermöglicht. Sobald eine Gasblase anstelle von Flüssigkeit durch den Leitungsabschnitt 32.9 strömt, ändert sich der Brechungsindex, was von den Sensoren zuverlässig detektiert werden kann und beispielsweise eine sofortige Sperrung des Hochdruckinjektors durch Verschluss des Ventilabschnitts D nach sich zieht.

Wesentlicher Vorteil der dargestellten Anordnung ist es, dass die Umlenkprismen 340,341 in einer definierten Position gleichermaßen wie die Leitungsabschnitte 32.1, 32.2, 32.3, 32.4, 32.5, 32.6, 32.7, 32.8, 32.9 auf der Leitungsfläche 300 des Hartteils 30 ausgebildet sein können und mit entsprechenden, ortsfest in der Kassettenhalterung 11 angeordneten Lichtquellen und Sensoren zusammenwirken, sobald die Steuerkassette 3 in der gewünschten korrekten Orientierung in die Kassettenhalterung 11 bzw. deren Aufnahmeraum 110 eingesetzt ist. Dies kann beispielsweise durch entsprechende, hier nicht dargestellte korrespondierende Stifte und Aufnahmebohrungen sichergestellt werden, die nur bei einer bestimmten Orientierung der Steuerkassette 3 in Eingriff kommen.

Da überdies die Austrittsöffnungen für den Lichtstrahl L1 und die Eintrittsöffnung für den Lichtstrahl L2 auf der Tür 112 oder Wand 113 des Aufnahmeraumes 110 liegen, lassen sich diese beispielsweise bei einem Wechsel der Steuerkassette 3 leicht reinigen.

Mit dem vorangehenden erläuterten Hochdruckinjektor ist es möglich, durch Integration sämtlicher Verbindungsleitungen sowie einer Einrichtung 33 zur Abscheidung von Gasblasen und einer Detektionseinrichtung 34 auf einer wechselbaren Steuerkassette 3 die Rüstzeiten des Hochdruckinjektors sowie den Aufwand für den Anschluss der Verbindungsleitungen enorm zu reduzieren und Fehlerquellen zuverlässig auszuschalten.

Darüber hinaus lassen sich die Steuerkassetten 3 nach Öffnen der dem Aufnahmeraum 110 zugeordneten Tür 112 leicht entnehmen und gegen eine neue Steuerkassette 3 austauschen, sodass den Anforderungen an die Ausführung der Verbindungsleitungen als Einwegteile Rechnung getragen werden kann.

Es versteht sich, dass anstelle der in den Figuren dargestellten Ausgestaltung des Hochdruckinjektors mit zwei Spritzen 2.1, 2.2 auch abweichende Ausführungsformen mit lediglich einer oder auch mehr als zwei Spritzen vorgesehen sein können.

Insbesondere bietet sich die Möglichkeit, die Spritzen 2.1, 2.2 gemeinsam mit der Steuerkassette 3 als Einheit vorzukonfektionieren und insoweit an den Anschlusselementen 36 vorzuverbinden und gegebenenfalls sogar die Spritzen 2.1 und 2.2 schon vorgefüllt vorzusehen, sodass auch keine Anschlüsse an den Anschlusselementen 35 mehr vorgenommen werden müssen. In einem solchen Fall genügt es, die Einheit aus Spritzen 2.1, 2.2 und Steuerkassette 3 in die kombinierte Kassettenhalterung 11 und Spritzenhalterung 10 einzulegen und den Patientenschlauch am Port 37 anzuschließen, um den Injektor betriebsbereit zu setzen. Sofern der Patientenschlauch bereits ebenfalls mit der Steuerkassette 3 vorverbunden ist, kann auch der letztgenannte Handgriff entfallen und die Bedienung auf das Einsetzen der vorverbundenen Einheit aus Steuerkassette 3 und Spritzen 2.1 sowie 2.2 nebst Patientenschlauch reduziert werden.

## Patentansprüche

1. Medizinischer Hochdruckinjektor für den Einsatz in Kombination mit einem CT- oder MRT-Gerät zur Injektion einer in einer Spritze (2.1, 2.2) bereitgestellten Flüssigkeit in einen Patienten mit hohem Druck von bis zu 83 bar, wobei der Injektor mindestens eine Spritzenhalterung (10) mit zugeordnetem Antrieb für die gehalterte Spritze (2.1, 2.2) sowie Verbindungsleitungen für die Überleitung der Flüssigkeit aus der Spritze (2.1, 2.2) in einen Patientenschlauch und/oder zur Befüllung der in der Spritzenhalterung 810) gehalterten Spritze (2.1, 2.2) aufweist, wobei die Verbindungsleitungen als wechselbare Steuerkassette (3) ausgebildet sind und der Injektor eine Kassettenhalterung (11) mit einem mit einer entsprechenden Tür (112) öffen- und schließbaren Aufnahmeraum (110) mit einer Wand (113) aufweist, in den die Steuerkassette (2) wechselbar einsetzbar ist, wobei die Dimensionierung des Aufnahmeraumes (110) so gewählt ist, dass die Steuerkassette (3) lediglich in einer vorgegebenen Orientierung in den Aufnahmeraum (110) eingesetzt werden kann und nach Verschließen der Tür (112) vorderseitig an der Tür (112) und rückseitig an der Wand (113) des Aufnahmeraumes 110 abgestützt wird, wobei die Steuerkassette (3) einen mindestens zweiteiligen Aufbau mit einem Hartteil (30) und mindestens einer Membran (31.1, 31.2) umfasst, wobei in eine als Leitungsfläche (300) ausgebildete Oberfläche des Hartteiles (30) Leitungsabschnitte (32.1, 32.2, 32.3, 32.4, 32.5, 32.6, 32.7, 32.8, 32.9) der Verbindungsleitungen eingebracht sind und wobei auf die Leitungsfläche (300) eine erste Membran (31.1) flüssigkeitsdicht aufgebracht ist welche die Leitungsabschnitte (32.1, 32.2, 32.3, 32.4, 32.5, 32.6, 32.7, 32.8, 32.9) im Hartteil (30) überdeckt und wobei in der der Leitungsfläche (300) gegenüberliegenden, als Ventilfläche (301) ausgebildeten Oberfläche des Hartteiles (30) Ventilabschnitte (A1, A2, B1, B2, C, D) der Verbindungsleitungen eingebracht sind und wobei die Ventilabschnitte (A1, A2, B1, B2, C, D) mittels einer auf die Ventilfläche (301) flüssigkeitsdicht aufgebrachten zweiten Membran (31.2) abgedeckt sind, wobei die Ventilabschnitte (A1, A2, B1, B2, C, D) mit den Leitungsabschnitten (32.1, 32.2, 32.3, 32.4, 32.5, 32.6, 32.7, 32.8, 32.9) über durch das Hartteil (30) von der Leitungsfläche (300) bis zur Ventilfläche (301) verlaufende Verbindungsbohrungen (4) kommunizieren und gemeinsam die Verbindungsleitungen ausbilden und wobei die Kassettenhalterung (11) mit, den Ventilabschnitten (A1, A2, B1, B2, C, D) der eingesetzten Steuerkassette (3) zugeordneten Stößeln (12) ausgerüstet ist, die in den Aufnahmeraum (110) vorschiebbar sind, um auf die zweite Membran (31.2) im Bereich des zugeordneten Ventilabschnittes (A1, A2, B1, B2, C, D) eine Druckkraft auszuüben und den Ventilabschnitt (A1, A2, B1, B2, C, D) zumindest teilweise zu verschließen.

2. Hochdruckinjektor nach Anspruch 1, **dadurch gekennzeichnet, dass** das Hartteil (30) aus einem spritzgegossenen Kunststoff und die erste und die zweite Membran (31.1, 31.2) aus einer mit dem Hartteil (30) verschweißbaren Kunststofffolie gebildet ist.

3. Hochdruckinjektor nach einem der Ansprüche 1 oder 2, **dadurch gekennzeichnet, dass** die in der Steuerkassette (3) ausgebildeten Verbindungsleitungen eine Einrichtung (33) zur Abscheidung von Gasblasen aus einer durch die Verbindungsleitungen strömenden Flüssigkeit umfassen.

4. Hochdruckinjektor nach Anspruch 3, **dadurch gekennzeichnet, dass** die Einrichtung (33) zur Abscheidung der Gasblasen einen kreisbogenförmig verlaufenden und mit Leitungsabschnitten (32.1, 32.2, 32.3, 32.4, 32.5, 32.6, 32.7, 32.8, 32.9) der Verbindungsleitungen kommunizierenden Kanal (330) umfasst, dessen Radius sich in einer vertikal verlaufenden Ebene erstreckt, wobei am höchsten Punkt der Einrichtung (33) eine Gasaustrittsöffnung (333) vorgesehen ist und der Kanal (330) sich in Strömungsrichtung der Flüssigkeit betrachtet verjüngt.

5. Hochdruckinjektor nach einem der Ansprüche 1 bis 4, **dadurch gekennzeichnet, dass** die Steuerkassette (3) eine optische Detektionseinrichtung (34) zum Detektieren von Gasblasen in einem Leitungsabschnitt (32.1, 32.2, 32.3, 32.4, 32.5, 32.6, 32.7, 32.8, 32.9) umfasst.

6. Hochdruckinjektor nach Anspruch 5, **dadurch gekennzeichnet, dass** die Detektionseinrichtung (34) zwei an gegenüberliegenden Positionen zu einem Leitungsabschnitt (32.1, 32.2, 32.3, 32.4, 32.5, 32.6, 32.7, 32.8, 32.9) angeordnete Umlenkprismen (340, 341) aufweist, wobei einem Umlenkprisma (340) eine in der Wandung des Aufnahmeraumes (110) angeordnete Lichtquelle zugeordnet ist und mittels des Umlenkprisma (340) der Lichtstrahl durch den Leitungsabschnitt (32.1, 32.2, 32.3, 32.4, 32.5, 32.6, 32.7, 32.8, 32.9) zum gegenüberliegenden Umlenkprisma (341) leitbar ist und dem Umlenkprisma (341) ein in der Wandung des Aufnahmeraumes (110) angeordneter Sensor zugeordnet ist.

7. Hochdruckinjektor nach einem der Ansprüche 1 bis 6, **dadurch gekennzeichnet, dass** die Steuerkassette (3) bereichsweise mit einer Heizeinrichtung für die durch die Verbindungsleitungen geleitete Flüssigkeit ausgerüstet ist.

8. Hochdruckinjektor nach Anspruch 7, **dadurch gekennzeichnet, dass** die Heizeinrichtung von einer bereichsweise auf der Steuerkassette (3) aufgebrachten strahlungsabsorbierenden Beschichtung und einer zugeordneten Lichtquelle im Aufnahmeraum (110) gebildet ist.

9. Hochdruckinjektor nach einem der Ansprüche 1 bis 8, **dadurch gekennzeichnet, dass** die Steuerkassette (3) und mindestens eine Spritze (2.1, 2.2) zu einer wechselbaren Einheit zusammengefasst und miteinander verbunden sind, die gemeinsam in den Aufnahmeraum (110) und die Spritzenhalterung (10) einsetzbar sind.

10. Hochdruckinjektor nach einem der Ansprüche 1 bis 9, **dadurch gekennzeichnet, dass** der Aufnahmeraum (110) und/oder die Steuerkassette (3) mit Mitteln zum lagerichtigen Einsetzen der Steuerkassette (3) in den Aufnahmeraum (110) ausgerüstet sind.

11. Hochdruckinjektor nach einem der Ansprüche 1 bis 10, **dadurch gekennzeichnet, dass** die Steuerkassette (3) mit einem Identifizierungsmittel ausgebildet ist, welches von Hochdruckinjektor bei in den Aufnahmeraum (110) eingesetzter Steuerkassette (3) auslesbar ist.

## Claims

1. High-pressure medical injector for use in combination with a CT or MRI appliance for injecting a liquid which is provided in a syringe (2.1, 2.2) into a patient at a high pressure of up to 83 bar, wherein the injector has at least one syringe holder (10) with an associated drive for the retained syringe (2.1, 2.2) and connection lines for transferring the liquid from the syringe (2.1, 2.2) into a patient hose and/or for filling the syringe (2.1, 2.2) which is retained in the syringe holder (10), wherein the connection lines are in the form of a replaceable control cartridge (3) and the injector has a cartridge holder (11) having a receiving space (110) which can be opened and closed with a corresponding door (112) and which has a wall (113), in which receiving space the control cartridge (2) can be inserted in a replaceable manner, wherein the sizing of the receiving space (110) is selected in such a manner that the control cartridge (3) can be inserted into the receiving space (110) only in a predetermined orientation and after the door (112) is closed is supported at the front on the door (112) and at the rear on the wall (113) of the receiving space (110), wherein the control cartridge (3) comprises an at least two-part construction having a hard portion (30) and at least one diaphragm (31.1, 31.2), wherein in a surface of the hard portion (30) in the form of a line face (300) line portions (32.1, 32.2, 32.3, 32.4, 32.5, 32.6, 32.7, 32.8, 32.9) of the connection lines are introduced and wherein a first diaphragm (31.1) is applied to the line face (300) in a fluid-tight manner and covers the line portions (32.1, 32.2, 32.3, 32.4, 32.5, 32.6, 32.7, 32.8, 32.9) in the hard portion (30), and wherein, in the surface, which is opposite the line face (300) and which is in the form of a valve face (301), of the hard portion (30), valve portions (A1, A2, B1, B2, C, D) of the connection lines are introduced and wherein the valve portions (A1, A2, B1, B2, C, D) are covered by means of a second diaphragm (31.2) which is applied to the valve face (301) in a fluid-tight manner, wherein the valve portions (A1, A2, B1, B2, C, D) communicate with the line portions (32.1, 32.2, 32.3, 32.4, 32.5, 32.6, 32.7, 32.8, 32.9) by means of connection holes (4) which extend through the hard portion (30) from the line face (300) up to the valve face (301) and together form the connection lines and wherein the cartridge holder (11) is provided with plungers (12) which are associated with the valve portions (A1, A2, B1, B2, C, D) of the control cartridge (3) used and which can be pushed forwards into the receiving space (110) in order to apply a pressure force to the second diaphragm (31.2) in the region of the associated valve portion (A1, A2, B1, B2, C, D) and at least partially to close the valve portion (A1, A2, B1, B2, C, D).

2. High-pressure injector according to claim 1, **characterized in that** the hard portion (30) is formed from an injection-molded plastics material and the first and second diaphragms (31.1, 31.2) are formed from a plastics material film which can be welded to the hard portion (30).

3. High-pressure injector according to either claim 1 or 2, **characterized in that** the connection lines which are formed in the control cartridge (3) comprise a apparatus (33) for separating gas bubbles from liquid flowing through the connection lines.

4. High-pressure injector according to claim 3, **characterized in that** the apparatus (33) for separating gas bubbles comprises a channel (330) which extends in the form of a circular arc and which communicates with line portions (32.1, 32.2, 32.3, 32.4, 32.5, 32.6, 32.7, 32.8, 32.9) of the connection lines, the channel radius extending in a vertically extending plane, wherein at the highest point of the apparatus (33) a gas outlet opening (333) is provided and the channel (330) tapers when viewed in the flow direction of the liquid.

5. High-pressure injector according to any one of claims 1 to 4, **characterized in that** the control cartridge (3) comprises a visual detection apparatus (34) for detecting gas bubbles in a line portion (32.1, 32.2, 32.3, 32.4, 32.5, 32.6, 32.7, 32.8, 32.9).

6. High-pressure injector according to claim 5, **characterized in that** the detection apparatus (34) has two deflection prisms (340, 341) which are arranged at opposing positions with respect to a line portion (32.1, 32.2, 32.3, 32.4, 32.5, 32.6, 32.7, 32.8, 32.9), wherein a deflection prism (340) is associated with a light source which is arranged in the wall of the receiving space (110) and by means of the deflection prism (340) the light beam can be directed through the line portion (32.1, 32.2, 32.3, 32.4, 32.5, 32.6, 32.7, 32.8, 32.9) to the opposing deflection prism (341) and a sensor which is arranged in the wall of the receiving space (110) is associated with the deflection prism (341).

7. High-pressure injector according to any one of claims 1 to 6, **characterized in that** the control cartridge (3) is provided in areas with a heating apparatus for the liquid which is directed through the connection lines.

8. High-pressure injector according to claim 7, **characterized in that** the heating apparatus is formed by a radiation-absorbing coating which is applied in areas to the control cartridge (3) and an associated light source in the receiving space (110).

9. High-pressure injector according to any one of claims 1 to 8, **characterized in that** the control cartridge (3) and at least one syringe (2.1, 2.2) are combined and connected to each other to form a replaceable unit which can be inserted together into the receiving space (110) and the syringe holder (10).

10. High-pressure injector according to any one of claims 1 to 9, **characterized in that** the receiving space (110) and/or the control cartridge (3) are provided with means for inserting the control cartridge (3) into the receiving space (110) in the correct position.

11. High-pressure injector according to any one of claims 1 to 10, **characterized in that** the control cartridge (3) is formed with an identification means which can be read by the high-pressure injector when the control cartridge (3) is inserted into the receiving space (110).

## Revendications

1. Injecteur haute pression médical destiné à être utilisé en combinaison avec un appareil CT ou MRT pour l'injection d'un liquide fourni dans une seringue (2.1, 2.2) à un patient à une pression élevée pouvant atteindre 83 bars, dans lequel l'injecteur présente au moins un support de seringue (10) avec un entraînement associé pour la seringue maintenue (2.1, 2.2) ainsi que des conduites de connexion pour le transfert du liquide de la seringue (2.1, 2.2) dans un tuyau de patient et/ou pour le remplissage de la seringue maintenue (2.1, 2.2) dans le support de seringue (810), dans lequel les conduites de connexion sont conçues en tant que cassette de commande interchangeable (3) et l'injecteur présente un support de cassette (11) avec un espace de logement (110) pouvant être ouvert et fermé avec une porte correspondante (112) avec une paroi (113), dans lequel la cassette de commande (2) peut être insérée de manière interchangeable, dans lequel le dimensionnement de l'espace de logement (110) est sélectionné de sorte que la cassette de commande (3) peut être insérée dans l'espace de logement (110) uniquement dans une orientation prédéfinie et, après fermeture de la porte (112), s'appuie à l'avant contre la porte (112) et à l'arrière contre la paroi (113) de l'espace de logement (110), dans lequel la cassette de commande (3) comprend une structure en au moins deux parties avec une partie dure (30) et au moins une membrane (31.1, 31.2), dans lequel des sections de conduite (32.1, 32.2, 32.3, 32.4, 32.5, 32.6, 32.7, 32.8, 32.9) des conduites de connexion sont introduites dans une surface de la partie dure (30) conçue en tant que surface de conduite (300) et dans lequel une première membrane (31.1) est appliquée de manière étanche aux liquides sur la surface de conduite (300), laquelle recouvre les sections de conduite (32.1, 32.2, 32.3, 32.4, 32.5, 32.6, 32.7, 32.8, 32.9) dans la partie dure (30) et dans lequel des sections de soupape (A1, A2, B1, B2, C, D) sont introduites dans la surface de la partie rigide (30) opposée à la surface de conduite (300) conçue en tant que surface de soupape (301) et dans lequel les sections de soupape (A1, A2, B1, B2, C, D) sont recouvertes par une seconde membrane (31.2) appliquée sur la surface de soupape (301) de manière étanche aux liquides, dans lequel les sections de soupape (A1, A2, B1, B2, C, D) communiquent avec les sections de conduite (32.1, 32.2, 32.3, 32.4, 32.5, 32.6, 32.7, 32.8, 32.9) par l'intermédiaire d'alésages de connexion (4) s'étendant à travers la partie dure (30) de la surface de conduite (300) jusqu'à la surface de soupape (301) et forment ensemble les conduites de connexion, et dans lequel le support de cassette (11) est équipé de poussoirs (12) associés aux sections de soupape (A1, A2, B1, B2, C, D) de la cassette de commande (3) insérée, qui peuvent être avancés dans l'espace de logement (110) pour exercer une force de pression sur la seconde membrane (31.2) au niveau de la section de soupape (A1, A2, B1, B2, C, D) et fermer au moins partiellement la section de soupape (A1, A2, B1, B2, C, D).

2. Injecteur haute pression selon la revendication 1, **caractérisé en ce que** la partie dure (30) est formée d'une matière plastique moulée par injection et les première et seconde membranes (31.1, 31.2) sont formées d'un film plastique soudable à la partie dure (30).

3. Injecteur haute pression selon l'une quelconque des revendications 1 ou 2, **caractérisé en ce que** les conduites de connexion formées dans la cassette de commande (3) comprennent un appareil (33) pour la séparation de bulles de gaz d'un liquide s'écoulant à travers les conduites de connexion.

4. Injecteur haute pression selon la revendication 3, **caractérisé en ce que** l'appareil (33) pour séparer les bulles de gaz comprend un canal (330) s'étendant en forme d'arc de cercle et communiquant avec des sections de conduite (32.1, 32.2, 32.3, 32.4, 32.5, 32.6, 32.7, 32.8, 32.9) des conduites de connexion, dont le rayon s'étend dans un plan vertical, dans lequel une ouverture de sortie de gaz (333) est prévue au point le plus haut de l'appareil (33) et le canal (330) s'effile dans le sens d'écoulement du liquide.

5. Injecteur haute pression selon l'une quelconque des revendications 1 à 4, **caractérisé en ce que** la cassette de commande (3) comprend un appareil de détection optique (34) pour détecter des bulles de gaz dans une section de conduite (32.1, 32.2, 32.3, 32.4, 32.5, 32.6, 32.7, 32.8, 32.9).

6. Injecteur haute pression selon la revendication 5, **caractérisé en ce que** l'appareil de détection (34) présente deux prismes de déviation (340, 341) disposés à des positions opposées par rapport à une section de conduite (32.1, 32.2, 32.3, 32.4, 32.5, 32.6, 32.7, 32.8, 32.9), dans lequel un prisme de déviation (340) est associé à une source lumineuse disposée dans la paroi de l'espace de logement (110) et, au moyen du prisme de déviation (340), le faisceau lumineux peut être guidé à travers la section de conduite (32.1, 32.2, 32.3, 32.4, 32.5, 32.6, 32.7, 32.8, 32.9) vers le prisme de déviation opposé (341) et un capteur disposé dans la paroi de l'espace de logement (110) est associé au prisme de déviation (341).

7. Injecteur haute pression selon l'une quelconque des revendications 1 à 6, **caractérisé en ce que** la cassette de commande (3) est équipée par zones d'un appareil de chauffage pour le liquide conduit par l'intermédiaire des conduites de connexion.

8. Injecteur haute pression selon la revendication 7, **caractérisé en ce que** l'appareil de chauffage est formé d'un revêtement absorbant le rayonnement appliqué par zones sur la cassette de commande (3) et d'une source lumineuse associée dans l'espace de logement (110).

9. Injecteur haute pression selon l'une quelconque des revendications 1 à 8, **caractérisé en ce que** la cassette de commande (3) et au moins une seringue (2.1, 2.2) sont regroupées et connectées entre elles pour former une unité interchangeable qui peut être insérée dans l'espace de logement (110) et le support de seringue (10).

10. Injecteur haute pression selon l'une quelconque des revendications 1 à 9, **caractérisé en ce que** l'espace de logement (110) et/ou la cassette de commande (3) sont équipés de moyens pour insérer la cassette de commande (3) dans l'espace de logement (110) dans la bonne position.

11. Injecteur haute pression selon l'une quelconque des revendications 1 à 10, **caractérisé en ce que** la cassette de commande (3) est conçue avec un moyen d'identification, lequel peut être lu par l'injecteur haute pression lorsque la cassette de commande (3) est insérée dans l'espace de logement (110).
